# EUROPÄISCHE PATENTSCHRIFT

(11) **EP 0 735 114 B1**
(45) Veröffentlichungstag und Bekanntmachung des Hinweises auf die Patenterteilung: **21.02.2001**
(21) Anmeldenummer: 96104562.2
(22) Anmeldetag: 22.03.1996
(51) Int. Cl.: C09C 1/00, C09D 7/12, C09D 11/00, C08K 9/02, C04B 33/14, C03C 4/02, A61K 7/00

(54) **Glanzpigmente auf Basis reduzierter titandioxidbeschichteter silikatischer Plättchen**
Brilliant pigments based on reduced silicate platelets coated with titanium dioxide
Pigments brilliants à base de plaquettes de silicate revêtues de dioxide de titane réduites

(30) Priorität: 30.03.1995 DE 19511696
(43) Veröffentlichungstag der Anmeldung: 02.10.1996
(73) Patentinhaber: BASF Aktiengesellschaft, 67063 Ludwigshafen (DE)
(72) Erfinder: Schmid, Raimund, Dr., 67435 Neustadt (DE); Kaliba, Claus, Dr., 67141 Neuhofen (DE); Mronga, Norbert, Dr., 69221 Dossenheim (DE); Ostertag, Werner, Dr., 67269 Grünstadt (DE); Schmidt, Helmut, 67574 Osthofen (DE); Gonzalez Gomez, Juan Antonio, Dr., 67063 Ludwigshafen (DE); Bidlingmaier, Hermann, 77654 Offenburg (DE); Ellinghoven, Raymond, 71672 Marbach (DE)

(56) Entgegenhaltungen:
- PATENT ABSTRACTS OF JAPAN vol. 9, no. 78 (C-274), 6.April 1985 & JP-A-59 212422 (SHISEIDO), 1.Dezember 1984,
- PATENT ABSTRACTS OF JAPAN vol. 10, no. 31 (C-327), 6.Februar 1986 & JP-A-60 184570 (SHISEIDO), 20.September 1985, & JP-A-60 184 570 (...)
- DATABASE WPI Week 8641 Derwent Publications Ltd., London, GB; AN 86-267354 XP002006649 & JP-A-61 192 749 (HINODE KAGAKU KOGYO) , 27.August 1986 & JP-A-61 192 749 (...)

## Beschreibung

Die vorliegende Erfindung betrifft neue Glanzpigmente, erhältlich durch Behandlung titandioxidbeschichteter silikatischer Plättchen mit einem Gasgemisch, das eine verdampfte organische Verbindung und Ammoniak enthält, bei 400 bis 900°C, wobei solche Glanzpigmente ausgenommen sind, die durch Behandlung titandioxidbeschichteter silikatischer Plättchen mit einer geometrischen Schichtdicke der Titandioxidbeschichtung von 10 bis 60 nm bei 800 bis 900°C erhältlich sind.

Weiterhin betrifft die Erfindung ein Verfahren zur Herstellung dieser Glanzpigmente sowie ihre Verwendung zum Einfärben von Lacken, Druckfarben, Tinten, Kunststoffen, Gläsern, keramischen Produkten und Zubereitungen der dekorativen Kosmetik.

Reduzierte titandioxidbeschichtete Glimmerpigmente, deren TiO₂-Beschichtung reduzierte Titanspezies (Oxidationszahl des Titans < 4 bis 2) enthält oder gänzlich in diese reduzierten Spezies umgewandelt ist, sind schon länger als "dunkle" Perlglanzpigmente für den blauen bis schwarzen Farbtonbereich bekannt und zeichnen sich durch gutes Deckvermögen, Farbstärke und Glanz aus.

Die dunklen Perlglanzpigmente lassen sich bekanntermaßen durch Behandlung von titandioxidbeschichteten Glimmerpigmenten mit reduzierenden Gasen bei Temperaturen von 600 bis 900°C herstellen.

Als reduzierende Gase werden dabei vorwiegend Ammoniak und Ammoniak/Stickstoff-Gemische eingesetzt. Die erhaltenen Produkte werden als blau, blauschwarz oder schwarz und die reduzierten Spezies als niedere Titanoxide wie Ti₃O₅, Ti₂O₃ bis zu TiO, Titanoxynitride sowie Titannitrid beschrieben (JP-A-164 653/1983, JP-A-126 468/1984, JP-A-184 570/1985, DE-A-34 33 657 und EP-A-332 071).

In der JP-A-192 749/1985 werden auch Wasserstoff und Propan als reduzierende Gase verwendet, wobei im Fall der Reduktion mit Propan schwarze, durch den Gehalt an Kohlenstoff schwach elektrisch leitende Pigmente entstehen.

Weiterhin wird in der EP-A-525 526 die Umsetzung von titandioxidbelegten Glimmerpigmenten mit Propan bei 850°C beschrieben, wobei neben der Abscheidung von Kohlenstoff auch das Titan reduziert werden soll.

Auch aus der US-A-3 087 827 ist die Belegung von titandioxidbeschichteten Glimmerpigmenten mit Kohlenstoff durch Pyrolyse von gasförmigen Kohlenwasserstoffen in Gegenwart der Glimmerpigmente oder, bei Verwendung von bei Raumtemperatur flüssigen Kohlenwasserstoffen, Bilden einer Pigment/Kohlenwasserstoff-Paste und anschließende Pyrolyse bei 700 bis 950°C bekannt.

Schließlich werden kohlenstoffhaltige Glimmerpigmente auch in den DE-A-25 57 796 und 41 41 049 beschrieben.

In der DE-A-25 57 796 werden zur Belegung titandioxidbeschichteter Glimmer mit einer weiteren, rußhaltigen Titandioxidschicht Glimmerpigment und Ruß in wäßriger Phase dispergiert, durch Hydrolyse von Titantetrachlorid wird eine rußhaltige Titanhydroxidschicht aufgefüllt, dann wird das Pigment getrocknet.

Aus der DE-A-41 41 049 ist die Beschichtung von titandioxidbelegtem Glimmer mit Kohlenstoff durch Aufbringen eines wäßrigen Zukkerfilms und anschließende Thermolyse bei 400 bis 500°C bekannt.

Auch diese mit Kohlenstoffschichten oder kohlenstoffhaltigen Schichten belegten Glimmerpigmente zeigen gutes Deckvermögen, Farbstärke und Glanz.

Die bekannten Pigmente können jedoch in mehrfacher Hinsicht nicht befriedigen. Je nach Herstellungsart liegen koloristische und/ oder anwendungstechnische Mängel vor.

So erhält man bei der Reduktion Interferenzfarben zeigender titandioxidbeschichteter Glimmerpigmente mit Ammoniak, Wasserstoff oder Kohlenwasserstoffen nur dann farbintensive Produkte, wenn die Interferenzfarbe des Glimmersubstrats mit der Absorptionsfarbe der Reduktionsprodukte übereinstimmt.

Da die reduzierten Titanspezies wie Ti₂O₃ blau gefärbt sind, resultieren nur bei Verwendung von TiO₂-Glimmerpigmenten mit blauer Interferenzfarbe farbintensive (blaue) Glanzpigmente.

Reduziert man dagegen z.B. ein rot reflektierendes Interferenzpigment, so entsteht durch die Mischung von Interferenz- und Absorptionsfarbe ein unattraktives Pigment mit brauner Körperfarbe. Allgemein werden bei Reduktion nicht blau reflektierender TiO₂-Glimmerpigmente stets unreine Farbtöne erhalten.

Außerdem zeigen insbesondere die mit Ammoniak oder Wasserstoff reduzierten titandioxidbeschichteten Glimmerpigmente in der Regel in Lacken mangelnde Schwitzwasserbeständigkeit, d.h. unter dem Einfluß von Wasserdampf treten irreversible Entfärbungen (Verweißlichungen) auf.

Kohlenstoffbeschichtete TiO₂-Glimmerpigmente, wie sie z.B. in der US-A-3 087 827 beschrieben sind, weisen zwar nicht die oben beschriebenen koloristischen Mängel auf, da üblicherweise im gesamten Farbtonbereich intensiv farbige Pigmente erhalten werden können, jedoch genügen diese Pigmente den heutigen Echtheitsanforderungen bei weitem nicht.

Glanzpigmente, bei denen Rußpigmente in die TiO₂-Schicht eingebaut sind, leiden häufig an mangelnder Abriebfestigkeit der Rußteilchen, die sich in einem Ausbluten des Rußes beim Dispergieren der Glanzpigmente in Lacken äußert. Außerdem zeigen diese Pigmente durch die Anwesenheit freier, nicht eingebauter Rußpartikel, die nur durch aufwendige Sedimentationsverfahren zu entfernen sind, oft Glanzverluste beim Einarbeiten in Lacken.

Der Erfindung lag daher die Aufgabe zugrunde, Pigmente bereitzustellen, welche die genannten Mängel nicht aufweisen.

Demgemäß wurden die eingangs definierten Glanzpigmente gefunden.

Außerdem wurde ein Verfahren zur Herstellung dieser Glanzpigmente gefunden, welches dadurch gekennzeichnet ist, daß man titandioxidbeschichtete silikatische Plättchen mit einem Gasgemisch, das eine verdampfte organische Verbindung und Ammoniak enthält, bei 400 bis 900°C behandelt.

Weiterhin wurde die Verwendung dieser Glanzpigmente zur Einfärbung von Lacken, Druckfarben, Tinten, Kunststoffen, Gläsern, keramischen Produkten und Zubereitungen der dekorativen Kosmetik gefunden.

Für die erfindungsgemäßen Glanzpigmente geeignete plättchenförmige silikatische Substrate sind insbesondere helle oder weiße Glimmer, wobei Schuppen von vorzugsweise naß vermahlenem Muskovit besonders bevorzugt sind. Selbstverständlich sind auch andere natürliche Glimmer, wie Phlogopit oder Biotit, künstliche Glimmer, Talk- und Glasschuppen geeignet.

Die Substratteilchen sind mit einer im wesentlichen aus Titandioxid bestehenden Schicht belegt, die als untergeordnete Bestandteile (im allgemeinen < 5 Gew.-%) weitere, vorzugsweise farblose, Metalloxide wie Zirkondioxid, Zinndioxid, Aluminiumoxid und Siliciumdioxid enthalten kann.

Derartige Pigmente sind allgemein bekannt und beispielsweise in den DE-A-14 67 468, DE-A-32 37 264 oder DE-A-20 09 566 beschrieben. Titandioxidbeschichtete Glimmerplättchen sind auch unter dem Namen Iriodin® (E. Merck, Darmstadt), Flonac® (Kemira Oy, Pori, Finnland) oder Mearlin® (Mearl Corporation, Ossining, New York) im Handel.

Die Größe der Substratteilchen ist an sich nicht kritisch und kann auf den jeweiligen Anwendungszweck abgestimmt werden. In der Regel haben die plättchenförmigen Teilchen mittlere größte Durchmesser von etwa 1 bis 200 µm, insbesondere etwa 5 bis 100 µm, und Dicken von etwa 0,1 bis 1 µm, insbesondere um etwa 0,5 µm. Ihre spezifische freie Oberfläche (BET) liegt üblicherweise bei 1 bis 15 m²/g, insbesondere 3 bis 12 m²/g.

Die Dicke der TiO₂-Schicht bestimmt den Farbton und beträgt je nach der gewünschten Interferenzfarbe in der Regel 40 bis 300 nm, vorzugsweise 50 bis 200 nm (geometrische Schichtdicke).

Die erfindungsgemäßen Glanzpigmente können vorteilhaft nach dem erfindungsgemäßen Herstellungsverfahren durch Behandlung mit einem Gasgemisch, das eine verdampfte organische Verbindung und Ammoniak enthält, bei 400 bis 900°C erhalten werden.

Als organische Verbindung eignen sich sowohl gasförmige Verbindungen als auch solche, die bei Raumtemperatur flüssig oder auch fest sind und verdampft werden können.

Bevorzugt sind dabei Kohlenwasserstoffe, besonders gesättigte aliphatische C₁-C₈-Kohlenwasserstoffe wie Pentan, Isopentan, Neopentan, Hexan, Heptan und Octan sowie deren verzweigte Isomere und ganz besonders Methan, Ethan, Propan und Butan und deren Isomere, auch ungesättigte aliphatische C₂-C₄-Kohlenwasserstoffe wie Ethen, Propen, Buten und deren Isomere und aromatische Kohlenwasserstoffe wie Benzol und Toluol.

Selbstverständlich können auch Gemische dieser Kohlenwasserstoffe, wie sie zum Beispiel im Erdgas vorliegen, eingesetzt werden.

Es können auch solche organischen Verbindungen zum Einsatz kommen, die nicht nur Kohlenstoff und Wasserstoff, sondern auch Sauerstoff und/oder Stickstoff enthalten, wobei jedoch je Kohlenstoffatom nicht mehr als ein Sauerstoff- und/oder Stickstoffatom im Molekül vorliegen sollte.

Beispiele für geeignete Verbindungen dieser Art sind C₁-C₅-Alkohole wie Methanol, Ethanol, Propanol und Isopropanol, C₃-C₇-Ketone wie Aceton, Di-C₁-C₂-alkylether wie Dimethyl- und Diethylether und cyclische Ether wie Tetrahydrofuran sowie Mono-C₁-C₅-alkylamine und Monoarylamine wie Methylamin, Ethylamin, Propylamin und Anilin, die auch allein, ohne Ammoniak eingesetzt werden können, wenn das Kohlenstoff/Stickstoff-Verhältnis der gewünschten Zusammensetzung des reduzierenden Gasgemisches entspricht. In der Regel sind die Amine jedoch schwerer zu handhaben.

Das in dem reduzierenden Gasgemisch vorliegende Volumenverhältnis der bevorzugten Komponenten Kohlenwasserstoff und Ammoniak kann stark variiert werden (prinzipiell im Bereich von 80:20 bis 1:99) und ist im Einzelfall an die gewünschten Produkteigenschaften anzupassen, die auch durch die Reaktionsdauer und die Reaktionstemperatur beeinflußt werden.

Kohlenstoffreichere und damit dunklere Glanzpigmente entstehen bevorzugt bei höheren Kohlenwasserstoffanteilen (in der Regel > 50 Vol.-%, bezogen auf das Gesamtvolumen der reduzierenden Gase), jedoch auch bei Verwendung höherer Alkane (z.B. Propan anstelle von Methan), bei längeren Reaktionszeiten (etwa 5 bis 10 h) und nicht zu hohen Reaktionstemperaturen (etwa 400 bis 800°C).

Kohlenstoffärmere, hellere Glanzpigmente werden umgekehrt insbesondere bei niedrigeren Kohlenwasserstoffanteilen (in der Regel < 50 Vol.-%, bezogen auf das Gesamtvolumen der reduzierenden Gase) sowie bei Verwendung niederer Alkane (insbesondere Methan oder Ethan), kürzeren Reaktionszeiten (etwa 1 bis 2 h) und höheren Reaktionstemperaturen (etwa 750 bis 900°C) erhalten.

Üblicherweise beträgt der Kohlenstoffgehalt der erfindungsgemäßen Glanzpigmente 0,1 bis 50 Gew.-%. Zur Herstellung farbiger Glanzpigmente ist ein Kohlenstoffgehalt von 0,2 bis 10 Gew.-%, bevorzugt 0,2 bis 5 Gew.-%, besonders geeignet. Bei Kohlenstoffgehalten > 10 Gew.-% ergeben sich in der Regel dunkle Glanzpigmente mit schwachem Farbschimmer und bei besonders hohen Kohlenstoffgehalten (> 25 Gew.-%) schwarze Glanzpigmente.

Bei den besonders geeigneten Kohlenwasserstoffen Methan, Ethan und Propan beträgt das Volumenverhältnis Ammoniak : Kohlenwasserstoff bevorzugt 4:1 bis 1:4, besonders bevorzugt 3:2 bis 2:3.

Zweckmäßigerweise wird das reduzierende Gasgemisch durch ein inertes Gas wie Stickstoff verdünnt. Dies empfiehlt sich insbesondere dann, wenn die organische Verbindung erst verdampft werden muß und mit Hilfe des inerten Gasstromes vorteilhaft in den Reaktionsraum überführt wird. Besonders günstig ist ein Stickstoffgehalt von 10 bis 60 Vol.-%, bezogen auf die gesamte Gasmenge. Man kann jedoch auch mit einem nur aus Kohlenwasserstoff und Ammoniak bestehenden Gasgemisch arbeiten.

Das erfindungsgemäße Verfahren wird in der Regel bei 400 bis 900°C, bevorzugt 600 bis 800°C, besonders bevorzugt 650 bis 750°C, durchgeführt.

Insbesondere bei Temperaturen ≤ 800°C werden besonders schwitzwasserbeständige (Note ≥ 4, Grauskala nach DIN 54001; Cleveland Humidity Test) und wetterechte (Note ≥ 4, Grauskala nach DIN 54001; Kurzbewitterungstest), damit auch lichtechte, Glanzpigmente erhalten, die gleichzeitig sehr farbintensiv und für den gesamten Farbbereich farbtonrein sind.

Durch die Einwirkung der organische Verbindung (besonders Kohlenwasserstoff) und Ammoniak enthaltenden Gasgemische wird auch bei den erfindungsgemäßen Glanzpigmenten (zumindest) teilweise die TiO₂-Schicht reduziert, wobei die eingangs beschriebenen reduzierten Spezies entstehen. Der Reduktionsgrad wird mit zunehmender Reaktionstemperatur und zunehmender Reaktionszeit größer, parallel dazu steigt auch der Stickstoffgehalt der Glanzpigmente, d.h. der Anteil an nitridischen Titanverbindungen.

Das erfindungsgemäße Verfahren kann sowohl kontinuierlich, z.B. in einem beheizten, inertisierten Drehrohrofen, in den das Glimmerpigment eingetragen und die reduzierenden Gase im Gemisch mit einem Inertgas eingeleitet werden, als auch diskontinuierlich, z.B. in einer beheizten, inertisierten Drehtrommel mit Gaszu- und -ableitung oder einem beheizten, inertisierten Wirbelbettreaktor, durchgeführt werden, wobei zweckmäßigerweise für eine allseitige Umspülung der Glimmerplättchen mit dem Reduktionsgas gesorgt wird, was im Drehrohrofen oder in der Drehtrommel vorzugsweise durch Stolperleisten ermöglicht wird.

Nach Beendigung der Reduktion, die im allgemeinen 1 bis 10 h, besonders 2 bis 7 h, dauert, wird das Glanzpigment vorzugsweise unter Inertgas abgekühlt. Gewünschtenfalls kann ein Desagglomerierungsschritt, beispielsweise in einem mit Schlagmessern ausgerüsteten Mischer, angeschlossen werden.

Die erfindungsgemäßen Glanzpigmente eignen sich vorteilhaft für viele Zwecke, wie zur Einfärbung von Kunststoffen, Gläsern, keramischen Produkten, Zubereitungen der dekorativen Kosmetik und insbesondere von Lacken, Tinten und Druckfarben, vor allem Sicherheitsdruckfarben (vgl. WO-A-94/13489, in der Glanzpigmente gezielt mit Farbpigmenten zur Herstellung von fälschungssicheren Wertschriften eingesetzt werden). Bei der Applikation im Druck sind alle industrieüblichen Druckverfahren, z.B. Siebdruck, Tiefdruck, Bronzierdruck, Flexodruck und Offsetdruck, geeignet.

Für diese Anwendungszwecke lassen sich die erfindungsgemäßen Glanzpigmente auch vorteilhaft in Abmischung mit transparenten und deckenden Weiß-, Bunt- und Schwarzpigmenten sowie auch herkömmlichen Glanzpigmenten verwenden.

Als Beispiele für geeignete Glanzpigmente seien ein- oder mehrfach metalloxidbeschichtete Glimmer- und Metallpigmente, unbeschichtete Metallpigmente auf Aluminiumbasis, schwarze Glanzpigmente, wie plättchenförmige Graphit- und Magnetitpigmente, und plättchenförmige Metalloxidpigmente, z.B. auf Basis von Eisen(III)oxid und Bismutoxychlorid, genannt.

Geeignete anorganische Pigmente sind z.B. Titanoxide, dotierte Titanoxide wie Nickeltitangelb, Eisenoxide, Bismutvanadate, farbige Spinelle, Chromat- und Cadmiumpigmente.

Als organische Pigmente eignen sich beispielsweise Monoazopigmente (z.B. Produkte, die sich von Acetessigarylidderivaten oder von β-Naphtholderivaten ableiten), verlackte Monoazofarbstoffe wie verlackte β-Hydroxynaphthoesäurefarbstoffe, Disazopigmente, kondensierte Disazopigmente, Isoindolinderivate, Derivate der Naphthalindicarbonsäure, Derivate der Perylentetracarbonsäure, Anthrachinonpigmente, Thioindigoderivate, Azomethinderivate, Chinacridone, Dioxazine, Pyrazolochinazolone, Indanthron- und Phthalocyaninpigmente.

### Beispiele

### Herstellung und Beurteilung von erfindungsgemäßen Glanzpigmenten

Die Herstellung der Glanzpigmente erfolgte in einem über einen Motor drehbaren und von einem zweischaligen Klappofen beheizten Einhalsrundkolben aus Quarzglas mit einer Gaszu- und -ableitung in der Drehachse.

### Anwendung im Lack

Zur Beurteilung der Koloristik der Pigmente im Lack wurden je 4 g der Pigmentproben in 96 g eines Polyester-Mischlackes mit 21 Gew.-% Festkörpergehalt eingerührt und 15 min unter 1500 Upm mit einem Propellerrührer dispergiert. Danach wurde der Basislackansatz auf eine Spritzviskosität von 18 sec im DIN-Becher 4 (DIN 53 211) eingestellt und auf ein nicht grundiertes Aluminiumblech gespritzt. Nach einer kurzen Ablüftzeit wurde naß in naß mit einem Einkomponenten-Klarlack auf Basis Acrylat/Melaminharz (47 Gew.-% Festkörpergehalt, eingestellt auf 23 Sec-DIN 4) überlackiert. Nach 30 minütigem Ablüften bei Raumtemperatur wurde 30 min bei 130°C eingebrannt.

Die Messung der CIELAB-Werte erfolgte anschließend mit einem Gonio-Spektralphotometer Multiflash M45 der Fa. Optronik (Berlin) bei einer Winkeldifferenz von 20°, 45° bzw. 75° zum Glanzwinkel. Die in der Tabelle angegebenen Farbwerte (L, a*, b*, HGD, C*) beziehen sich auf die Normlichtart D65. Dabei entspricht L der Helligkeit, a* dem Rot- bzw. Grünanteil und b* dem Blau- bzw. Gelbanteil. HGD ist der Farbwinkel [°] und C* das Chroma.

### Prüfung auf Schwitzwasserbeständigkeit

Die Beurteilung des Schwitzwasserverhaltens der Pigmente im Lack erfolgte nach dem sog. Cleveland Humidity Test. Die mit dem pigmentierten Basislack lackierten Bleche (jeweils sowohl ein nicht grundiertes Aluminiumblech als auch ein Karosserieblech mit folgendem Lackaufbau: Bonderblech (zinkphosphatiertes Stahlblech) / kathodische Tauchlackierung / Grundierung auf Polyester/Acetobutyratbasis) wurden in einem Cleveland Condensing Humidity Cabinet (Condensation Tester Q·C·T der Fa. The Q-Panel Company; Cleveland, Ohio, USA) plaziert. Das mit vollentsalztem Wasser gefüllte Bad wurde auf 70°C Wasserbadtemperatur eingestellt, und die Bleche wurden 24 h lang bei 100 % relativer Luftfeuchte kontinuierlich betaut.

Nach Abschluß der Schwitzwasserbelastung wurden die Bleche abgetrocknet und unverzüglich bezüglich einer Farbänderung nach der Grauskala gemäß DIN 54 001 (entspricht ISO 105) beurteilt. Nach dieser Skala werden farblich unveränderte Lackierungen mit 5 und vollständig weiß angelaufene Lackierungen mit 1 bewertet.

### Prüfung der Wetterechtheit

Zur Beurteilung der Wetterechtheit wurden die lackierten Bleche (nicht grundiertes Aluminiumblech) bis zu 80 d lang in einem Xenotest 1200 - Gerät der Fa. Heraeus (Hanau) gemäß DIN 53387-1-B-X ("Künstliches Bewittern oder Bestrahlen in Geräten - Beanspruchung durch gefilterte Xenonbogenstrahlung", April 1989) belastet.

Als Strahler werden dabei 3 Xenonstrahler NXe 4500 mit einer Bestrahlungsstärke von 65 bis 75 W/m² (Fa. Heraeus) benutzt. Der Strahlerwechsel erfolgt im Rotationsverfahren alle 500 Betriebsstunden, wobei im Endeffekt jeder Strahler nach 1500 Betriebsstunden ausgetauscht wird.

Die Expositionsbedingungen sind wie folgt festgelegt: Wendelaufbetrieb durch Besprühen mit VE-Wasser; Beregnungszyklus: 18 min naß/102 min trocken; relative Luftfeuchte in der Naßphase ca. 95 % und in der Trockenphase 60 bis 70 %; Schwarztafeltemperatur: 63 bis 67°C.

Die bewitterten Bleche wurden anschließend wie oben nach der Grauskala gemäß DIN 54 001 (ISO 105) beurteilt.

### Beispiel 1

75 g eines titandioxidbeschichteten Glimmerpigmentes mit roter Reflexionsfarbe (Iriodin® 221 Rutil Feinrot; E. Merck, Darmstadt) wurden in der oben beschriebenen Apparatur durch einstündiges Überleiten von 15 l/h Stickstoff inertisiert. Nach Erhitzen auf 700°C wurde 2 h ein Gemisch aus 5 l/h Ammoniak, 5 l/h Propan und 5 l/h Stickstoff durch die Apparatur geleitet. Das anschließende Abkühlen auf Raumtemperatur erfolgte unter 15 l/h Stickstoff.

Es wurde ein intensiv rot gefärbtes Pigment (Kohlenstoffgehalt von 2,1 Gew.-%, Stickstoffgehalt < 0,2 Gew.-%) mit sehr guter Schwitzwasserbeständigkeit (Bewertung 4 - 5) und sehr guter Wetterechtheit (80 d Bewitterung, Bewertung 5) erhalten.

### Vergleichsbeispiel V1

Es wurde analog Beispiel 1 vorgegangen, jedoch erfolgte keine Propanzufuhr.

Das Pigment verfärbte sich kaum und behielt seine weiße Körperfarbe (Stickstoffgehalt < 0,2 Gew.-%).

Schwitzwasserbeständigkeit und Lichtechtheit wurden aufgrund der mangelhaften Koloristik nicht geprüft.

### Vergleichsbeispiel V2

Es wurde analog Beispiel 1 vorgegangen, jedoch erfolgte keine Ammoniakzufuhr.

Das erhaltene Pigment war intensiv rot gefärbt (Kohlenstoffgehalt 1,1 Gew.-%), war jedoch weder schwitzwasserbeständig (Bewertung 2) noch wetterecht (20 d Bewitterung, Bewertung 1 - 2).

### Vergleichsbeispiel V3

75 g des Glimmerpigments aus Beispiel 1 wurden 2 h bei 850°C mit 75 l/h Ammoniak reduziert.

Das Pigment verfärbte sich von weiß nach schmutzig braun. Die rote Interferenzfarbe ist nicht mehr zu beobachten (Stickstoffgehalt 1,7 Gew.-%).

Schwitzwasserbeständigkeit und Wetterechtheit wurden aufgrund der mangelhaften Koloristik nicht geprüft.

**Tabelle**

| Koloristische Daten | | | | | |
|---|---|---|---|---|---|
| Beispiel | HGD | C* | L | a* | b* |
| | | | | | |

| Meßwinkel 20° | | | | | |
|---|---|---|---|---|---|
| 1 | 335,9 | 32,2 | 48,3 | 29,3 | -13,1 |
| V1 | 12,3 | 23,2 | 83,4 | 22,6 | 5,0 |
| V2 | 342,8 | 36,2 | 55,0 | 34,6 | -10,7 |
| V3 | 55,8 | 23,3 | 59,8 | 13,1 | 19,2 |

| Meßwinkel 45° | | | | | |
|---|---|---|---|---|---|
| 1 | 336,1 | 14,0 | 18,6 | 12,8 | -5,7 |
| V1 | 107,1 | 12,2 | 63,9 | -3,6 | 11,7 |
| V2 | 345,1 | 15,6 | 24,6 | 15,1 | -4,0 |
| V3 | 30,1 | 7,1 | 23,3 | 6,1 | 3,5 |

| Meßwinkel 75° | | | | | |
|---|---|---|---|---|---|
| 1 | 348,1 | 4,9 | 12,0 | 4,8 | -1,0 |
| V1 | 128,4 | 15,5 | 59,9 | -9,7 | 12,2 |
| V2 | 1,8 | 5,7 | 17,1 | 5,7 | 0,2 |
| V3 | 303,9 | 3,8 | 14,5 | 2,1 | -3,1 |

## Patentansprüche

1. Glanzpigmente, erhältlich durch Behandlung titandioxidbeschichteter silikatischer Plättchen mit einem Gasgemisch, das eine verdampfte organische Verbindung und Ammoniak enthält, bei 400 bis 900°C, wobei solche Glanzpigmente ausgenommen sind, die durch Behandlung titandioxidbeschichteter silikatischer Plättchen mit einer geometrischen Schichtdicke der Titandioxidbeschichtung von 10 bis 60 nm bei 800 bis 900°C erhältlich sind.

2. Glanzpigmente nach Anspruch 1, bei denen als organische Verbindung Methan, Ethan, Propan, n-Butan und/oder iso-Butan eingesetzt werden.

3. Glanzpigmente nach Anspruch 1 oder 2, bei denen die silikatischen Plättchen Glimmerplättchen sind.

4. Verfahren zur Herstellung von Glanzpigmenten gemäß den Ansprüchen 1 bis 3, dadurch gekennzeichnet, daß man titandioxidbeschichtete silikatische Plättchen mit einem Gasgemisch, das eine verdampfte organische Verbindung und Ammoniak enthält, bei 400 bis 900°C behandelt.

5. Verwendung von Glanzpigmenten gemäß den Ansprüchen 1 bis 3 zur Einfärbung von Lacken, Druckfarben, Tinten, Kunststoffen, Gläsern, keramischen Produkten und Zubereitungen der dekorativen Kosmetik.

## Claims

1. Luster pigments obtainable by treating titania-coated silicatic platelets at from 400 to 900°C with a gas mixture comprising a vaporized organic compound and ammonia, excluding luster pigments obtainable by treating titania-coated silicatic platelets with a geometric layer thickness of from 10 to 60 nm for the titania coating at from 800 to 900°C.

2. Luster pigments as claimed in claim 1, where for the organic compound used is methane, ethane, propane, n-butane and/or isobutane.

3. Luster pigments as claimed in claim 1 or 2, wherein the silicatic platelets are mica platelets.

4. A process for producing the luster pigments as claimed in any of claims 1 to 3, characterized in that titania-coated silicatic platelets are treated at from 400 to 900°C with a gas mixture comprising a vaporized organic compound and ammonia.

5. The use of luster pigments as claimed in any of claims 1 to 3 for coloring paints, inks, including printing inks, plastics, glasses, ceramic products and decorative cosmetic preparations.

## Revendications

1. Pigments brillants, pouvant être obtenus en traitant des plaquettes de silicate revêtues de dioxyde de titane avec un mélange gazeux qui contient un composé organique vaporisé et de l'ammoniac, à une température de 400°C à 900°C, où l'on exclut les pigments brillants que l'on peut obtenir en traitant des plaquettes de silicate revêtues de dioxyde de titane ayant une épaisseur de couche géométrique du revêtement de dioxyde de titane de 10 à 60 nm, à une température de 800°C à 900°C.

2. Pigments brillants selon la revendication 1, dans lesquels on met en oeuvre le méthane, l'éthane, le propane, le n-butane et/ou l'isobutane en tant que composé organique.

3. Pigments brillants selon la revendication 1 ou 2, dans lesquels les plaquettes de silicate sont des plaquettes de mica.

4. Procédé de préparation de pigments brillants selon les revendications 1 à 3, caractérisé en ce que l'on traite les plaquettes de silicate revêtues de dioxyde de titane avec un mélange gazeux qui contient un composé organique vaporisé et de l'ammoniac, à des températures de 400°C à 900°C.

5. Utilisation des pigments brillants selon les revendications 1 à 3, pour la coloration de laques, d'encres d'impression, d'encres, de matières plastiques, de verres, de produits céramiques et de préparations de la cosmétique décorative.
